# EUROPEAN PATENT APPLICATION

(11) **EP 4 283 282 A1**
(43) Date of publication of application: **29.11.2023**
(21) Application number: 22742638.4
(22) Date of filing: 20.01.2022
(51) Int. Cl.: G01N 21/64

(54) **AUTOFLUORESCENCE QUENCHING DEVICE**

(30) Priority: 22.01.2021 JP 2021008882
(71) Applicant: Toho University, Tokyo 143-8540 (JP); Nepa Gene Co., Ltd., Ichikawa-shi, Chiba 272-0114 (JP)
(72) Inventor: TSUNEOKA, Yousuke, Tokyo 143-8540 (JP); FUNATO, Hiromasa, Tokyo 143-8540 (JP)
(74) Representative: J A Kemp LLP
(86) International application number: PCT/JP2022/001941
(87) International publication number: WO 2022/158516

(57) **Abstract**

Disclosed is an autofluorescence quenching device that: quenches only autofluorescence without quenching fluorescence that is originally desired to be observed in a tissue section in a short time; does not emit noise in various wavelength regions; is capable of controlling a temperature increase; can be used repeatedly with a simple operation; and can stably quenches autofluorescence. The autofluorescence quenching device for a sample includes: a sample placement unit; and an irradiation unit mounted on the sample placement unit. The sample placement unit includes: a flat plate; one or more sample placement recesses each in which a sample is to be placed and which are provided in the flat plate; and a cooling means for cooling the sample placement recesses. The irradiation unit includes one or a plurality of white LED illuminations placed above the respective sample placement recesses.

## Description

### TECHNICAL FIELD

The present invention relates to an autofluorescence quenching device that quenches the autofluorescence from a biological sample or the like.

### BACKGROUND ART

The development of optical machines and staining techniques has made it possible to perform an analysis approaching the essence of the mechanism of action of biological phenomena, and the research has progressed to the level of cells and sub-cellular levels. In the fluorescence imaging technology using a fluorescent probe, in order to obtain accurate macroscopic and visible features of organs and tissues of living organisms, it is necessary to perform the imaging considering phenomena and characteristics such as fluorescence quenching, fluorescence intensity, photobleaching, and fluorescence background. If there is no method of quenching or masking fluorescence derived from autofluorescence substances such as lipofuscin derived from living organisms, or fluorescence derived from tissue fixative treatment, it is substantially impossible that fluorescence staining signals are uniformly acquired and imaged without depending on the state of tissues and cells. Therefore, in the field of life science, especially in histology (pathology), there is a high demand for a technique that enables efficient quenching of autofluorescence.

Here, the techniques for quenching the autofluorescence in the prior art can be categorized into the chemically quenching technique using a dedicated reagent and the physically quenching technique by irradiating a tissue section using a light source such as a mercury lamp.

With the chemically quenching technique, there are many remaining drawbacks as follows: although the auto fluorescence disappears, the fluorescence originally desired to be observed also disappears; noise increases in another wavelength region; the cost depends on the number of samples; and the like. There is not much track record in paper reports using these.

On the other hand, in the physically quenching technique too, there are many remaining drawbacks including that it takes 4 to 48 hours for quenching, although the autofluorescence disappears; irreversible denaturation of tissue sections due to the temperature rise during irradiation time; and the like. Since it is necessary to avoid these drawbacks (Non-Patent Document 1), after all, there is not much track record in paper reports using the physically quenching technique.

### CITATION LIST

### NON-PATENT LITERATURE

Non-Patent Document 1: Doung H and Han M 2013 A multispectral LED array for the reduction of background autofluorescence in brain tissue. Journal of neuroscience methods, 220: 46-54

### SUMMARY OF INVENTION

### TECHNICAL PROBLEM

The present invention has been made in view of the circumstances in the above prior art, and an object of the present invention is to provide a technique that: quenches only the autofluorescence without quenching the fluorescence originally desired to be observed in the tissue section in a short time; emits no noise even in various wavelength regions; and is also capable of controlling temperature rise, so as to provide a technique that can be used repeatedly with a simple operation, and is capable of stably quenching the autofluorescence.

### SOLUTION TO PROBLEM

As a result of intensive research, the present inventors have found that by using a white LED illumination as an irradiation source, only the autofluorescence is quenched without quenching the fluorescence originally desired to be observed in the tissue section in a short time, such that noise is not emitted in various wavelength regions. Furthermore, an autofluorescence quenching device was conceived that uses the white LED illumination as the irradiation source, which can suppress the temperature rise and can be used repeatedly with a simple operation, to complete the present invention.

That is, the present invention provides the following:
(1) An autofluorescence quenching device for a sample, comprising:
   a sample placement unit; and an irradiation unit mounted on the sample placement unit,
   wherein the sample placement unit comprises a flat plate, one or more sample placement recesses each in which a sample is to be placed and which are provided in the flat plate, and a cooling means for cooling the sample placement recesses, and
   the irradiation unit comprises one or a plurality of white LED illuminations placed above the respective sample placement recesses.
(2) The autofluorescence quenching device of (1), wherein the cooling means is a heat sink and/or a cooling fan provided under the flat plate.
(3) The autofluorescence quenching device of any one of (1) to (3), wherein the irradiation unit comprises a second flat plate, and the white LED illumination is disposed on the second flat plate.
(4) The autofluorescence quenching device of (3), wherein the irradiation unit further comprises a second cooling means.
(5) The autofluorescence quenching device of (4), wherein the second cooling means is a heat sink and/or a cooling fan provided on the second flat plate.
(6) The autofluorescence quenching device of any one of (3) to (5), wherein the irradiation unit is mounted on the sample placement unit via ribs, and a space is provided between the flat plate and the second flat plate by the ribs.

### ADVANTAGEOUS EFFECTS OF INVENTION

According to the present invention, a technique is provided that: quenches only the autofluorescence without quenching the fluorescence originally desired to be observed in the tissue section in a short time; emits no noise in various wavelength regions; and is also capable of controlling temperature rise, so that an autofluorescence quenching device was provided that can be used repeatedly with a simple operation, and is capable of stably quenching the autofluorescence.

### BRIEF DESCRIPTION OF DRAWINGS

FIG. 1 is a schematic perspective view of a preferred embodiment of an autofluorescence quenching device of the present invention.
FIG. 2 is a partial perspective cross-sectional view of the embodiment shown in FIG. 1.
FIG. 3 is a schematic perspective view showing an irradiation unit turned upside down in the embodiment shown in FIG. 1.
FIG. 4 is a schematic perspective view showing a sample placement unit of the embodiment shown in FIG. 1.

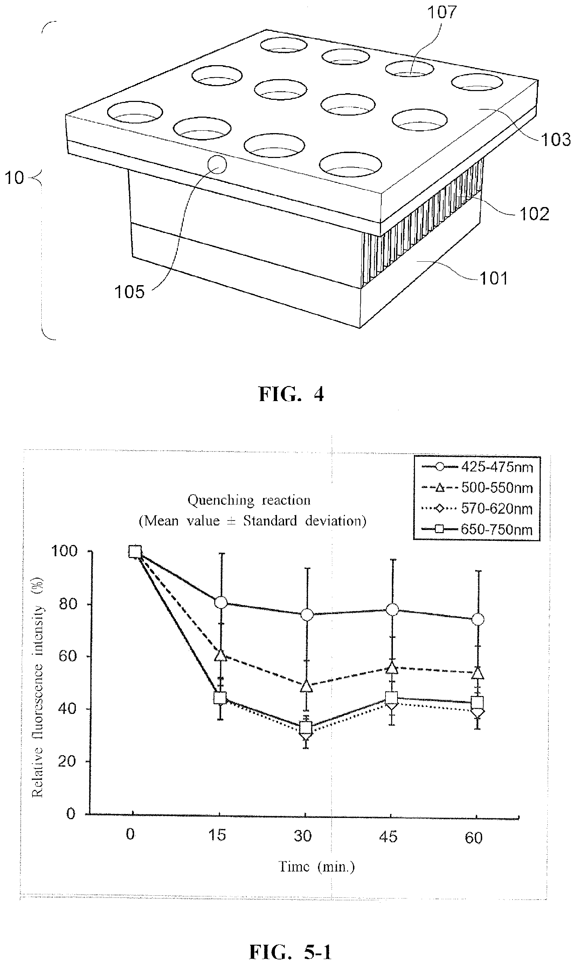

FIG. 5-1 is a figure which shows the relationship between quenching time and relative degree of quenching in each wavelength region in Example 1; wherein in each wavelength region of 425-475nm (blue), 500-550nm (green), 570-620nm (red) and 650-750 nm (infrared), the quantity of light in autofluorescence seen at places where cellular tissues exist in the mouse cerebral cortex is shown; the quantity of light at 0 minute of light irradiation was taken as 100%, and the quantity of light at the lumen of a blood vessel or the like where no mouse tissue exists was taken as 0%; and numerical values are shown by mean values and standard deviations.
FIG. 5-2 shows the results of the same experiment as FIG. 5-1 and shows the numerical values at the maximum values.

### DESCRIPTION OF EMBODIMENTS

Hereinafter, a preferred embodiment of the present invention will be described with reference to the drawings.

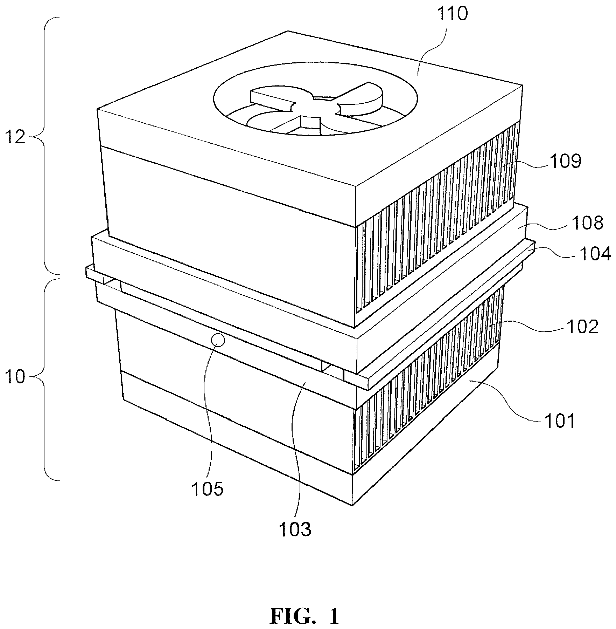

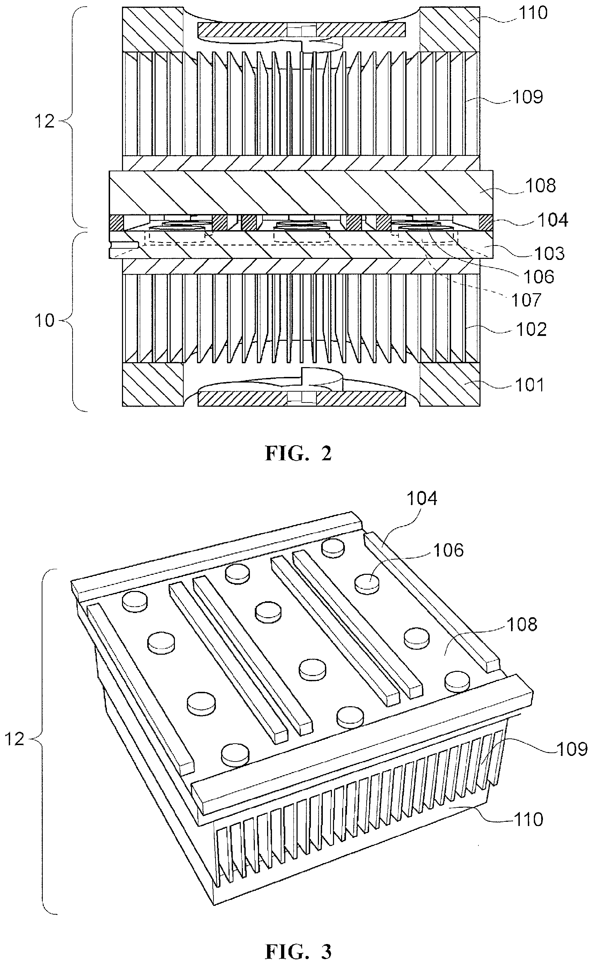

FIG. 1 is a schematic perspective view of a preferred embodiment of an autofluorescence quenching device of the present invention; FIG. 2 is a partial perspective cross-sectional view of the embodiment shown in FIG. 1; FIG. 3 is a schematic perspective view showing an irradiation unit turned upside down in the embodiment shown in FIG. 1; FIG. 4 is a schematic perspective view showing a sample placement unit of the embodiment shown in FIG. 1.

As shown in FIG. 1, the autofluorescence quenching device of the present invention comprises a sample placement unit 10 and an irradiation unit 12 mounted on the sample placement unit 10. As well shown in FIG. 4, the sample placement unit 10 comprises a flat plate 103. The material of the flat plate 103 is not particularly limited, but is preferably a metal that easily dissipates heat, and examples thereof include silver, copper, aluminum, and the like. In addition, the upper surface of the flat plate 103 may be mirrored so as to easily reflect light. Alternatively, a method of suppressing heat generation may also be employed, which uses a material having low heat absorption such as glass or acrylic resin so as to irradiate light in a liquid. A plurality of sample arrangement recesses 107 are formed in the flat plate 103. Although only one sample placement recess 107 may be provided, a plurality thereof is preferably provided because they enable to process a plurality of samples simultaneously. In the illustrated embodiment, the sample placement recess 107 is formed in a cylindrical shape, but the shape is not particularly limited. For example, it can be formed in a shape in which a glass slide, a culture dish, a cover glass, or the like can be placed as is. In cases where the sample placement recess 107 is formed in the cylindrical shape, the size thereof is not particularly limited, but the diameter of the circle thereof is usually about 10 mm to 20 mm and the depth is about 2 mm to 8 mm.

A heat sink 102 that is a cooling means for the sample placement recess 107 is disposed under the flat plate 103, and a fan 101 (see FIG. 2) is further disposed under the heat sink. Note that as the cooling means, only one of the heat sink 102 and the fan 101 may be sufficiently provided, but it is preferable to provide both of them because the cooling performance is enhanced. As the cooling means, in addition to the heat sink and fan, it is also possible to use a Peltier cooling unit, a cold water chiller unit, and the like.

On the other hand, the irradiation unit 12 comprises a second flat plate 108. As well shown in FIG. 3, a plurality of white LED illuminations 106 are disposed on the second flat plate 108. The material of the second flat plate 108 is not particularly limited, but is preferably a metal that easily dissipates heat, and examples thereof include silver, copper, aluminum, and the like. In addition, the upper surface of the second flat plate 108 may be mirror-finished so as to easily reflect light. The plurality of white LED illuminations 106 are disposed corresponding to the plurality of sample placement recesses 107, and provided at positions such that the plurality of white LED illuminations are disposed directly above the sample placement recesses 107, respectively, when the irradiation unit 12 is mounted on the sample placement unit 10. The distance between the lower surface of the white LED illumination 106 and the bottom surface of the sample placement recess 107 is usually about 3 mm to 20 mm, preferably about 4 mm to 10 mm. The white LED illumination 106 is connected to a power supply (not shown), and will be lit in use. The intensity of the white LED illumination in use is set appropriately, and usually set to about 1000 lumens to 8000 lumens, preferably about 1500 lumens to 5000 lumens. It should be noted that the output of the white LED illumination is preferably set to be adjustable in 0-1000 W, preferably 0-500 W, more preferably 0-400 W; the setting resolution for the output is preferably a unit of 100 W, more preferably a unit of 10 W. Additionally, the white LED illumination usually emits white light by a combination of a blue LED and a yellow phosphor that is a complementary color thereof. The blue LED preferably has a dominant wavelength (peak wavelength) of about 450 nm to 520 nm, and more preferably, 470 nm to 500 nm.

Ribs 104 are disposed between the second flat plate 108 and the flat plate 103 of the sample placement unit 10 (see FIG. 3). The ribs 104 function as a spacer for ensuring a distance between the flat plate 103 of the sample placement unit 10 and the second flat plate 108 of the irradiation unit 12. This allows a desired value for a distance between the lower surface of the white LED illumination 106 and the bottom surface of the sample placement recess 107.

A second heat sink 109 that is a cooling means is disposed on the second flat plate 108, and further a second fan 110 that is also a cooling means is disposed thereon. Note that the irradiation unit 12 may not have the cooling means, but its presence is preferable because it can further suppress the temperature rise of the sample. Although one of the heat sink 109 and the fan 110 may be used, the presence of these two is preferable because it can increase cooling performance thereof.

Furthermore, a thermometer probe insertion port 105 may be formed on the flat plate 103, and a thermometer probe may be inserted thereinto so that the temperature in the flat plate 1 03 (close to the temperature of the sample placement recess 107) can be measured. In this case, there may be provided: a control means for controlling the drive current of the irradiation unit 12; a control means for controlling the drive current of the cooling means in the sample placement unit 12; a temperature detection means for detecting the temperature of the sample placement unit 12; a set means for setting a threshold value of a predetermined temperature considered; a judging means for judging the presence or absence of abnormality occurrence based on the temperature detection means and the threshold value set in the set means; a stop means for automatically stopping the drive current based on the judgment by the judging means. This enables to prevent the sample from being damaged due to undesired heat.

In operation, a sample such as a tissue section of an organism is put in the sample arrangement recess 107. Note that the sample such as the tissue section of the organism is not particularly limited, and a species of the organism and a tissue material are not limited as long as a size thereof is prepared to be put in the sample arrangement recess 107. Next, the irradiation unit 12 is mounted on the sample placement unit 10. Thereby, each white LED illumination 106 is disposed directly above each sample placement recess 107. In this state, each white LED illumination 106 is turned on. The lighting time can be set as appropriate, and it is usually about 15 minutes to 1 hour. The above operation can quench the auto fluorescence of the sample. Additionally, in operation, the entire autofluorescence quenching device may be attached to a shaker and irradiation may be performed with shaking. In this case, since the sample in the sample placement recess 107 moves relatively to some extent in the recess, there is an advantage that the irradiation to the sample can be made more uniform.

Hereinafter, the present invention will be specifically described based on examples. However, the present invention is not limited to the following examples.

### Example 1

All animal experiments were conducted in accordance with the guidelines of the Toho University Animal Care and User Committee, and the procedure was approved by the Toho University Animal Care and User Committee (approval number 19-51-405). Mating pairs of C57/BL6 mice were purchased from Japan SLC Inc. and CLEA Japan Inc. Mice were raised in a breeding colony within the university under a 12-hour light-dark cycle and controlled conditions of 23 ± 2 °C and 55% ± 5% humidity. Brains of male mice at 20-30 weeks of age were sampled. The mice were deeply anesthetized by intraperitoneal administration of pentobarbital sodium of 50 mg/ml and transcardially perfusion-fixed by 4% paraformaldehyde phosphate buffer. The removed brain was fixed overnight at 4°C with 4% formaldehyde and subjected to anti-freezing treatment by infiltration in a 30% sucrose solution for two days; the resultant was sealed in Surgipath (trade name: Manufactured by Leica Biosystem) and then stored at -80 °C until use. Brain slices were formed as cryosections with a thickness of 40 µM.

An autofluorescence quenching device according to the embodiment of the above-described present invention shown in FIGS. 1 to 4 was prepared. Note that the dominant wavelength of the blue LED constituting the white LED illumination 106 was 475 nm. After washing the sections with phosphate buffer solution, the sections were moved using a brush to the sample placement recesses 107 filled with a buffer solution; the one not irradiated with light, and the ones irradiated with light for each of 15 minutes, 30 minutes, 45 minutes, and 60 minutes were prepared. The light irradiation was performed uniformly under the condition at 24 W for each LED. The LED has an intensity of 4022 lumens at 32 W; the sample placement recess 107 had a diameter of 15 mm and a depth of 4 mm; the distance from the LED to the bottom surface of the sample placement recess 107 was 5.5 mm. During the light irradiation, uniform light irradiation conditions were obtained by shaking the tissue sections at a speed of 60 rpm with a shaking machine, and the temperature of the sample placement recess 107 was continuously cooled to be 37 °C or lower.

Fluorescent photographs of stained tissue sections were taken with respect to the cerebral cortex by a Nikon Eclipe Ni microscope equipped with an A1R confocal detection system. The laser wavelengths used for fluorescence excitation were 405 nm, 488 nm, 560 nm and 640 nm, and the images of the fluorescence were acquired at wavelengths of 425-475 nm (blue), 500-550 nm (green), 570-620 nm (red) and 650-750 nm (infrared). The acquired images were analyzed in luminous intensity by Imaged.

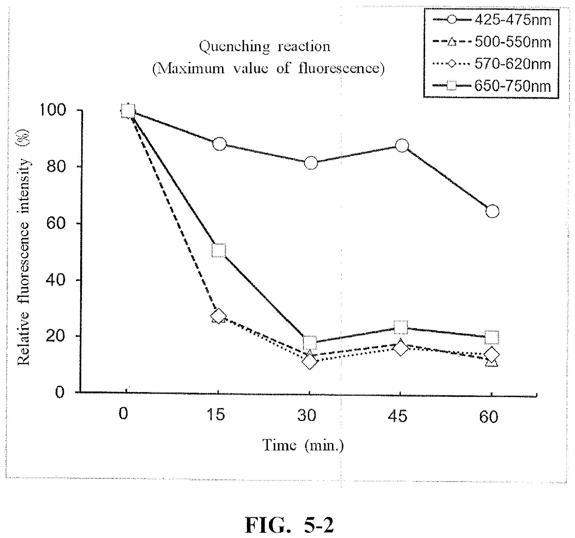

In the tissue sections that were not irradiated with light, fluorescence in the form of granules such as lipofuscin granules was clearly observed, especially remarkable at infrared wavelengths. The fluorescence in the form of granules gradually attenuated from the start of the light irradiation, and was no longer observed after 30 minutes. That is, in each wavelength region of 425-475nm (blue), 500-550nm (green), 570-620nm (red) and 650-750 nm (infrared), fluorescent tissue sections of unstained sections in the mouse cerebral cortex were observed. Before the light irradiation, granular signals were observed over all wavelengths, but almost disappeared after 15 minutes from the start of the light irradiation, and the granular signals were no longer recognized after 30 minutes. In addition, not only the fluorescence in the form of granules but also the autofluorescence observed in the entire tissue section was quenched by the light irradiation, and after 30 minutes, resulting in 50% or less from the start time in the wavelengths other than blue. In blue fluorescence, fading of about 20% was recognized from the start time (Figure 5-1). Similarly, the maximum value of the fluorescence in the section also largely decreased, resulting in about 20% from the starting time at wavelengths other than blue after 30 minutes (Figure 5-2).

### REFERENCE SIGNS LIST

- 10: sample placement unit
- 12: irradiation unit
- 101: fan
- 102: heat sink
- 103: flat plate
- 104: rib
- 105: thermometer probe insertion port
- 106: white LED illumination
- 107: sample placement recess
- 108: second flat plate
- 109: second heat sink
- 110: second fan

## Claims

1. An autofluorescence quenching device for a sample, comprising:
a sample placement unit; and
an irradiation unit mounted on the sample placement unit,
wherein the sample placement unit comprises a flat plate, one or more sample placement recesses each in which a sample is to be placed and which are provided in the flat plate, and a cooling means for cooling the sample placement recesses, and
the irradiation unit comprises one or a plurality of white LED illuminations placed above the respective sample placement recesses.

2. The autofluorescence quenching device of claim 1, wherein the cooling means is a heat sink and/or a cooling fan provided under the flat plate.

3. The autofluorescence quenching device of claim 1 or 2, wherein the irradiation unit comprises a second flat plate, and the white LED illumination is disposed on the second flat plate.

4. The autofluorescence quenching device of claim 3, wherein the irradiation unit further comprises a second cooling means.

5. The autofluorescence quenching device of claim 4, wherein the second cooling means is a heat sink and/or a cooling fan provided on the second flat plate.

6. The auto fluorescence quenching device of any one of claims 3 to 5, wherein the irradiation unit is mounted on the sample placement unit via ribs, and a space is provided between the flat plate and the second flat plate by the ribs.
